# EUROPEAN PATENT SPECIFICATION

(11) **EP 2 482 864 B1**
(45) Date of publication and mention of the grant of the patent: **14.11.2018**
(21) Application number: 09764656.6
(22) Date of filing: 24.11.2009
(51) Int. Cl.: A61L 27/34, A61L 27/54, A61L 17/00, A61L 17/14, A61L 24/00, A61L 29/08, A61L 29/16, A61L 31/10, A61L 31/16

(54) **ANTIMICROBIAL/ANTIBACTERIAL MEDICAL DEVICES COATED WITH TRADITIONAL CHINESE MEDICINES**
ANTIMIKROBIELLE/ANTIBAKTERIELLE MEDIZINISCHE VORRICHTUNGEN MIT EINER BESCHICHTUNG AUS TRADITIONELLEN CHINESISCHEN ARZNEIMITTELN
DISPOSITIFS MÉDICAUX ANTIMICROBIENS / ANTIBACTÉRIENS REVÊTUS AU MOYEN DE MÉDICAMENTS CHINOIS TRADITIONNELS

(30) Priority: 29.09.2009 US 569571
(43) Date of publication of application: 08.08.2012
(60) Divisional application: 18182837.7
(73) Proprietor: Ethicon, Inc., Somerville, New Jersey 08876-0151 (US)
(72) Inventor: POKROPINSKI, Henry, South River NJ 08882 (US); FISCHER, Jerome, A., Warren NJ 07059 (US); JONN, Jerry, Beijing 100016 (US); WANG, Huimin, Beijing 100016 (CN); ROTHENBURGER, Stephen, Neshanic Station NJ 08853 (US); MING, Xintian, Bridgewater NJ 08807 (US)
(74) Representative: Kirsch, Susan Edith
(86) International application number: PCT/US2009/065799
(87) International publication number: WO 2011/040936

(56) References cited:
- EP-A1- 1 889 608
- WO-A1-02/47701
- DATABASE WPI Week 199645 Thomson Scientific, London, GB; AN 1996-450041 XP002613178, & JP 8 224270 A (UETANI K) 3 September 1996 (1996-09-03)
- DATABASE WPI Week 200926 Thomson Scientific, London, GB; AN 2009-B51669 XP002613179, & CN 101 297 827 A (WANG) 5 November 2008 (2008-11-05)
- DATABASE WPI Week 200566 Thomson Scientific, London, GB; AN 2005-640188 XP002613180, & CN 1 616 057 A (ZHANG P) 18 May 2005 (2005-05-18)

## Description

This disclosure relates to medical devices comprising compositions derived from traditional Chinese medicines.

Each year, patients undergo a vast number of surgical procedures in the United States. Current data shows about twenty-seven million procedures are performed per year. Post-operative or surgical site infections ("SSIs") occur in approximately two to three percent of all cases. This amounts to more than 675,000 SSIs each year.

In a surgical setting, when a medical device is used, a risk of infection may be created. The risk of infection dramatically increases for invasive or implantable medical devices, such as intravenous catheters, arterial grafts, intrathecal or intracerebral shunts and prosthetic devices, which create a portal of entry for pathogens while in intimate contact with body tissues and fluids. The occurrence of surgical site infections may be often associated with bacteria that colonize on the medical device. For example, during a surgical procedure, bacteria from the surrounding atmosphere may enter the surgical site and attach to the medical device. Bacteria can use the implanted medical device as a pathway to surrounding tissue. Such bacterial colonization on the medical device may lead to infection and morbidity and mortality to the patient. Another adverse aspect concerns the formation of biofilms. Bacteria living in a biofilm usually have significantly different properties from free-floating bacteria of the same species, due to the dense and protected environment of the film. This environment may provide an increased resistance to antibiotics, as the dense extracellular matrix and the outer layer of cells protect the interior of the bacterial community. In some cases antibiotic resistance can be increased by 1000 fold.

A number of methods for reducing the risk of infection associated with invasive or implantable medical devices have been developed that incorporate antimicrobial agents into the medical devices. Such devices desirably provide effective levels of antimicrobial agent while the device is being used.

Traditional Chinese Medicine, also known as TCM, includes a range of traditional medical practices originating in China. TCM practices include such treatments as herbal medicine, acupuncture, dietary therapy, and both Tui na and Shiatsu massage. Qigong and Taijiquan are also closely associated with TCM. Although well accepted in the mainstream of medical care throughout East Asia, broadly speaking, TCM is often considered to be an alternative medical system in much of the western world.

Houttuynia cordata, also known as herba houttuyniae and, in Japan, as "dokudami," is a perennial plant widely distributed in Asia, including Japan, Taiwan, China, Himalayan and Java. Houttuynia cordata is a flowering plant that grows in moist, shady places and is the sole species in the genus Houttuynia. In Chinese, it is literally known as "fishy-smelling herb." Houttuynia is also used in traditional Chinese medicine (TCM). The beverage dokudami cha, in Japanese, literally "Houttuynia cordata tea" is an infusion made from Houttuynia cordata leaves, oolong tea leaves, and Job's Tears.

U.S. Patent Publication No. 2002/0031559 proposes a suppository for treating human ailments comprising at least one herb and a suppository vehicle. A method of treating undesired symptoms from allergic rhinitis, sinusitis, nasal congestion, nasal dripping, nasal polyps, infections, fevers, coughs, spasms, dizziness, convulsions in a human is also proposed that uses a suppository having herbs. Methods of producing, administrating and formulating herbal medicines in the form of suppositories to treat human aliments and disease are also proposed. A suppository that may include Houttuynia cordata as an ingredient is proposed.

U.S. Patent Publication No. 2006/0264347 discloses an antimicrobial composition comprising a cationic surfactant derived from the condensation of fatty acids and esterified dibasic amino acids, such as lauric arginate, and an iodinated sulfone compound such as diiodomethyl-p-tolylsulfone. The composition may be used as a stand-alone antimicrobial formulation, or in combination with medical articles or medical devices.

U.S. Patent No. 7,485,327 proposes a composition comprising Melissa leaf extract that is said to inhibit angiogenesis and matrix metalloproteinase activity. The Melissa leaf extract is said to inhibit angiogenesis and activity of matrix metalloproteinase, so that it can be applied to treat or prevent diseases related to angiogenesis and matrix metalloproteinase. The composition comprising Melissa leaf extract may also comprise more than one component of other anti-angiogenic, anti-cancer, anti-inflammatory and anti-aging components. This particular composition comprising Melissa leaf extract can be used for pharmaceutical, dietetic and/or cosmetic purposes. U.S. Patent No. 7,485,327 proposes using Houttuynia cordata extract as an ingredient with Melissa leaf which is an antibacterial. The composition may be administered via an implant.

Chinese Publication No. 2005-10046735 reportedly proposes an eye preparation containing sodium new houttuyfonate and a process for preparation, wherein the preparation comprises sodium new houttuyfonate as the active constituent and pharmaceutically acceptable auxiliary materials, and can be prepared into various eye preparations including eye drops, gels, turbid liquors, microspheres, microemulsions, implants and effervescent tablets..

WO9850087 proposes coated medical devices adapted to pass through narrow body openings such as catheters. The coatings provided impart durability to the catheter without appreciably adding to the thickness of the catheter and without decreasing the hoop tensile strength of the catheter.

WO0126708 proposes polymeric valves, valve devices, machines and instruments. The proposed devices include implantable devices with a sufficiently long lifetime that are responsive to the patient's therapeutic requirements and deliver a certain amount of a drug in response to a biological stimulus.

Despite these advances in the art, it would be beneficial to incorporate an antimicrobial/antibacterial composition into an invasive or implantable medical device to reduce the risk of infection. Further, it would be desirable to provide an antimicrobial/antibacterial composition derived from one or more traditional Chinese medicines, which exhibits activity upon contact with fluids in the human body.

EP-A-1889608 discloses extracts prepared by extracting *Houttuynia cordata* in water. These aqueous extracts are then used to form hydrogel compositions that are coated onto suitable substrates and used for the treatment of atopic dermatitis.

JP-A-8224270 discloses absorbent products comprising a deodorizing layer of plant fibers of houttuynia cordata.

The present invention provides a medical device comprising an antimicrobial composition, wherein said medical device is in the form of a suture anchor device, suture, catheter, staple, stent, surgical tack, clips, plate and screw, adhesion prevention barrier, or tissue adhesive, and characterized in that said antimicrobial composition comprises an antimicrobial agent selected from the group of extract of Houttuynia cordata, sodium houttuyfonate, sodium new houttuyfonate and mixtures thereof.

In one form, the antimicrobial composition comprises sodium houttuyfonate or sodium new houttuyfonate having a mean particle size of less than 100 µm.

In another form, the antimicrobial composition comprises sodium houttuyfonate or sodium new houttuyfonate having a mean particle size of less than 50 µm.

In yet another form, the antimicrobial composition comprises sodium houttuyfonate or sodium new houttuyfonate having a mean particle size of less than 40µm.

In still yet another form, the antimicrobial composition comprises sodium houttuyfonate or sodium new houttuyfonate having a mean particle size of less than 40µm and a standard deviation particle size of less than 30µm.

Suitably, the antimicrobial composition is in the form of a coating on at least one surface of the medical device and said coating further comprises a polymeric film forming material.

In these embodiments, the polymeric film forming material suitably comprises a biocompatible, biodegradable polymer, copolymer hydrogel or blends thereof.

Suitably, the polymeric film forming material is selected from aliphatic polyesters, poly(amino acids), copoly(ether-esters), polyalkylenes oxalates, polyamides, poly(ethylene glycol), poly(iminocarbonates), polyorthoesters, polyoxaesters, polyamidoesters, polyoxaesters containing amine groups, poly(anhydrides), polyphosphazene, polysaccharide gels and copolymers and blends thereof.

Suitably, the polymeric film forming material is selected from homopolymers and copolymers of lactide, glycolide, epsilon-caprolactone, para-dioxanone, trimethylene carbonate, alkyl derivatives of trimethylene carbonate, monoglyceride polyesters, carboxymethyl cellulose hydrogels and blends thereof.

In one form, the polymeric film forming material is selected from homopolymers of lactide (PLA) and homopolymers of glycolide (PGA).

In another form, the polymeric film forming material is selected from copolymers of PLA and PGA.

In still another form, the antimicrobial coating composition includes calcium stearate.

in another aspect, the present invention provides a method of making the claimed antimicrobial medical device, the method comprising the step of applying an antimicrobial coating composition to a medical device, the antimicrobial coating composition including a polymeric film forming material and from 1% to 15% w/w of an antimicrobial agent selected from the group of sodium houttuyfonate, sodium new houttuyfonate and mixtures thereof.

In one form, the antimicrobial medical device is packaged and sterilized.

Several advantages and benefits of one or more aspects disclosed herein include: excellent in vitro antimicrobial efficacy against SSI pathogens, ability to evenly and homogenously distribute the disclosed coating formulations, controlled release antimicrobial properties, and the increased efficacy and stability of the disclosed antimicrobial coating formulations. Additionally, medical devices coated with the disclosed antimicrobial coating formulations exhibit excellent stability after sterilization. Moreover, potential side effects are limited or non-existent due to the nature of the antimicrobial agents and coating formulations disclosed herein.
FIG. 1 presents a graphical representation of the effect of particle size on sodium houttuyfonate concentration for a sodium houttuyfonate coated suture; and
FIG. 2 presents a graphical representation of the effect of particle size on zone of inhibition for a sodium houttuyfonate coated suture.

As used herein, the singular forms "a," "an," and "the" include the plural reference unless the context clearly dictates otherwise.

By biodegradable, it is meant that a polymer may be degraded or otherwise broken down in the body such that the components of the degraded polymer may be absorbed by or otherwise passed from the body.

Each of the following terms: "includes," "including," "has," "'having," "comprises," and "comprising," and, their linguistic or grammatical variants, derivatives, and/or conjugates, as used herein, means "including, but not limited to."

Disclosed herein but not being part of the invention, is an antimicrobial composition for coating a medical device.

The antimicrobial composition includes a polymeric film forming material and a traditional Chinese medicine (TCM) antimicrobial agent comprising a material selected from the group of extract of Houttuynia cordata, sodium houttuyfonate and sodium new houttuyfonate and mixtures thereof.

As indicated above, Houttuynia cordata is widely distributed in Asia, including Japan, Taiwan, China, Himalayan and Java. Houttuynia cordata is a flowering perennial plant that grows in moist, shady places and is the sole species in the genus Houttuynia. Houttuynia is also used in TCM. The beverage dokudami cha, in Japanese, literally "Houttuynia cordata tea" is an infusion made from houttuynia cordata leaves, oolong tea leaves, and Job's Tears. Like low striped bamboo extract, Houttuynia cordata extract is obtained by a low temperature/high pressure extraction method.

Herba houttuyniae is the whole fresh herb or the dried part of Houttuynia cordata. The key antimicrobial component is Houttuynin or decanoyl acetaldehyde, which is unstable and prone to polymerize. The current synthesized, sodium sulfite derivatives, known as sodium houttuyfonate or sodium new houttuyfonate, are more stable and, thus useful in medical applications.

Houttuynin has been shown to have a marked suppressive effect on various bacteria and viruses. The double anti-infective activities in terms of prevention and treatment of secondary infections and against viral infections have been found to be beyond those of general antibiotics. Tests in vitro have demonstrated that Houttuynin has a marked inhibitory effect on Micrococcus catarrhalis, Staphylococcus aureus, Bacillus influenzae, pneumococcus, Escherichia coli, Bacillus dysenteria, Bacillus proteus, Bacillus diphtheriae, and mycobacteriae, and also a strong effect on Bacillus typhi, and Leptopira.

Houttuynin apozem has been found to have an inhibitory action on the influenza virus (Asia-China origin) and epidemic hemorrhagic fevers virus (EHFV) in vitro, and could delay the cytopathogenic effect of Orphan virus strain 11 (ECHO 11). The fixed oil component of houttuynin by peritoneal injection has been shown to have a prophylactic effect on mice that were infected by influenza virus FM1, and the drug via mouth or nose also offered a degree of protection.

The clinical application of Houttuynia cordata dates back to the Tang Dynasty, when the herb was documented in the book "Chinese Materia Medica."

Currently available Houttuynia cordata preparations include water apozem, houttuynin tablets, houttuynin injections, syrup for acute bronchitis, eliminating phlegm capsule, Huang Long cough with asthma powder preparation for infusion, and mango antitussive and others.

Sodium houttuyfonate, C₁₂H₂₃O₅SNa, also known as decanoyl acetaldehyde sodium sulfide, has a molecular weight of 302.36 and is a white or almost-white, needle-like crystalline powder that possesses a slight odor. Suitable sources for sodium houttuyfonate include Hubei Yuanhe Organic Foodstuffs Co., Ltd, Jinkou Industrial Area, Zhifang Street, Wuhan, Zhejiang, China 430074 and Second Pharma Co., Ltd., Hangzhou Gulf Fine Chemical Zone, Shangyu, Zhejiang, China 312369.

Sodium new houttuyfonate, C₁₄H₂₇NaO₅S, is available in needle-shaped crystals or a crystal powder and is white in color. It is available from Hangzhou HETD Pharmaceutical & Chemical Co., Ltd., No. 8, Yi Road, Xi Yuan Xi Hu Industrial Park, Sandun Hangzhou, Zhejiang, China 310030 and Linyi Furui Fine Chemical Co., Ltd shandong china, linyi, Shandong, China 251500. Sodium new houttuyfonate is a scale-like or crystalline powder, possessing a somewhat disagreeable odor. It is easily soluble in hot water, slightly soluble in water and ethanol, and almost insoluble in chloroform and benzene. It is easily soluble in a sodium hydroxide solution.

It has been discovered that the beneficial antimicrobial effect of TCM's, such as sodium houttuyfonate, may be enhanced through a grinding operation aimed at reducing the mean particle size of the crystalline material.

Commercial grade sodium houttuyfonate has a particle size distribution ranging from approximately 1000 µm to 4 µm, with a mean of approximately 145 µm. This widely variable particle size distribution results from the longitudinal and axial measurement of the needle-like crystals. The particle size and geometry of sodium houttuyfonate produces aggregates, resulting in an uneven coating distribution when applied to a substrate. As such, it was found that the geometry of the raw material and its particle size and distribution require modification and refinement to produce an acceptable coating. Particle size reduction can be accomplished through various well-known processing techniques, such as a ball mill or jet mill. A ball mill reduces particle size by means of mechanically crushing the material through collision with hardened media (i.e. balls). A jet mill operates on the principal of accelerating particles through a stream of air, gas or steam, where the resultant collisions cause fracturing. These processing techniques fracture the sodium houttuyfonate crystals and produce particles with improved symmetry and significantly narrower particle size distribution. The particle size distribution can additionally be selectively narrowed even further through classification.

As such, in one form, the antimicrobial composition comprises sodium houttuyfonate or sodium new houttuyfonate or mixtures thereof having a mean particle size of less than 100 µm. In another form, the antimicrobial composition comprises sodium houttuyfonate or sodium new houttuyfonate having a mean particle size of less than 50 µm. In yet another form, the antimicrobial composition comprises sodium houttuyfonate or sodium new houttuyfonate having a mean particle size of less than 40 µm. In still yet another form, the antimicrobial composition comprises sodium houttuyfonate or sodium new houttuyfonate having a mean particle size of less than 40 µm and a standard deviation particle size of less than 30 µm.

The antimicrobial compositions disclosed herein may advantageously serve as coating compositions, providing a vehicle for delivering the antimicrobial agent to the surface of the medical device. As those skilled in the art will recognize, coatings are used conventionally in the manufacture of certain medical devices, such as, for example, absorbable and non-absorbable multifilament sutures. Examples of medical devices, as well as coatings that may be applied thereto, may be found in U.S. Patent Nos. 4,201,216, 4,027,676, 4,105,034, 4,126,221, 4,185,637, 3,839,297, 6,260,699, 5,230,424, 5,555,976, 5,868,244, and 5,972,008. As disclosed in U.S. Patent No. 4,201,216, the coating composition may include a film-forming polymer and a substantially water-insoluble salt of a C₆ or higher fatty acid. As another example, an absorbable coating composition that may be used for an absorbable medical device may include poly(alkylene oxylates) wherein the alkylene moieties are derived from C₆ or mixtures of C₄ to C₁₂ diols, which is applied to a medical device from a solvent solution, as disclosed in U.S. Patent No. 4,105,034. The compositions disclosed may include a polymer or co-polymer, which may include lactide and glycolide, as a binding agent. The compositions may also include calcium stearate, as a lubricant. Medical devices not conventionally employing a coating in the manufacturing process, however, also may be coated with an antimicrobial composition comprising a TCM antimicrobial agent.

Examples of suitable biocompatible, biodegradable polymers that could be used according to the present invention include, without limitation, polymers selected from the group consisting of aliphatic polyesters, poly(amino acids), copoly(ether-esters), polyalkylenes oxalates, polyamides, poly(ethylene glycol), poly(iminocarbonates), polyorthoesters, polyoxaesters, polyamidoesters, polyoxaesters containing amine groups, poly(anhydrides), polyphosphazenes, biopolymers, and copolymers and blends thereof.

Aliphatic polyesters having utility include, but are not limited to, homopolymers and copolymers of lactide (which includes lactic acid, D-,L- and meso lactide), glycolide (including glycolic acid), epsilon-caprolactone, para-dioxanone (1,4-dioxan-2-one), trimethylene carbonate (1,3-dioxan-2-one), alkyl derivatives of trimethylene carbonate, monoglyceride polyesters, and polymer blends thereof.

Preferred polymers utilized in the invention comprise homopolymers of lactide (PLA) and homopolymers of glycolide (PGA). More preferred are copolymers of PLA and PGA (PLGA), such copolymers comprising from about 80 to about 99 mole percent PLA.

In one form, the polymeric film forming material comprises a biocompatible, biodegradable polymer, copolymer or blends thereof. The polymeric film forming material may be selected from aliphatic polyesters, poly(amino acids), copoly(ether-esters), polyalkylenes oxalates, polyamides, poly(ethylene glycol), poly(iminocarbonates), polyorthoesters, polyoxaesters, polyamidoesters, polyoxaesters containing amine groups, poly(anhydrides), polyphosphazene, polysaccharide gels and copolymers and blends thereof.

In another form, the polymeric film forming material may be selected from homopolymers and copolymers of lactide, glycolide, epsilon-caprolactone, para-dioxanone, trimethylene carbonate, alkyl derivatives of trimethylene carbonate, monoglyceride polyesters, carboxymethyl cellulose hydrogels and blends thereof. The polymeric film forming material may be selected from homopolymers of lactide (PLA) and homopolymers of glycolide (PGA). In one form, the polymeric film forming material is selected from copolymers of PLA and PGA.

In a further aspect, the antimicrobial composition may optionally contain other components that improve the antimicrobial effectiveness of the composition, or that otherwise serve as active agents for other benefits. These components include, but are not limited to, additional antimicrobials, additional salts, any other excipients or active ingredients that provide the compositions with beneficial properties or enhance the antimicrobial activity of the compositions. Such components include, but are not limited to, antimicrobial agents such as triclosan, triclocarban, 2-phenoxyethanol, chlorhexidine salts, hexetidine and cetylpyridinium salts; antibiotics; and other active ingredients.

The antimicrobial compositions described herein may be used to coat at least one surface of the medical device disclosed in claim 1. Additionally, they can be a part of the coating that contains the antimicrobial composition described herein. These coatings may comprise either a single layer or multiple layers. In another form, the antimicrobial composition may also be applied to a preformed article or part of an article of manufacture as a coating. The coated article may be produced, for example, by dipping the article into the composition, coextruding the article, wire coating the article, or spraying the article with the composition and then drying the coated article.

The antimicrobial compositions described herein are used alone or in combination with other polymer coatings to provide advantageous properties to the surface of the substrate. These compositions can also be used, to deliver pharmaceutical agents that, for example, are antiinfective, anticoagulants, improve healing, are antiviral, antifungal, antithrombogenic or impart other properties to coated substrates.

The antimicrobial compositions may also be used to inhibit algae, fungal, mollusk, or microbial growth on surfaces. The antimicrobial compositions described herein may also used as herbicides, insecticides, antifogging agents, diagnostic agents, screening agents, and antifoulants.

Coating dispersions may be prepared preferably by initially solubilizing all soluble compounds in an organic solvent or solvent blend and then adding insoluble compounds to the solution. The dispersion is created by combining the aforementioned compounds in a vessel that minimizes solvent evaporation and then blending with a mechanical mixer. The mixer must impart sufficient energy to create a homogeneous dispersion or suspension without allowing aggregation of the insoluble compounds. Suitable devices are high speed blade mixers, homogenizers, sonicators, vortexers and other mixing devices commonly employed that produce, dispersions, suspensions or emulsions.

The TCM antimicrobial agents disclosed herein may be present in the coating composition in a concentration of from 1 to 15% w/w, or from 1.5 to 7.5% w/w, or from 2.0 to 5.0% w/w. In one form, the TCM antimicrobial agents disclosed herein are present in a concentration of about 2.5% w/w. In another form, the TCM antimicrobial agents disclosed herein are present in a concentration of about 5% w/w.

The coating of medical devices with TCM antimicrobial agent dispersions, such as sodium houttuyfonate dispersions should be performed in a manner which precisely controls the amount and distribution of the coating applied to the medical device. The coating operation can be performed by a traditional dip and wipe method as described in patent U.S. Patent No. 5,817,129, where the substrate is passed through a vessel containing the suspension or dispersion, then passed over a set of wheels to remove excess coating and sent through a drying system to remove the carrier solvent. Coatings can be also applied to medical devices by spraying, drip coating, tank dipping, web coating and other coating methodologies well know to the industry.

In another aspect, disclosed herein is an article of manufacture that is a medical device that comprises the antimicrobial compositions described herein. The medical device is selected from a suture anchor device, suture, catheter, staple, stent, surgical tack, clips, plate and screw, adhesion prevention barrier, and tissue adhesive. The medical device may be coated with one or more of the antimicrobial compositions disclosed herein. In one form, the medical device may be coated or impregnated with the antimicrobial composition by dipping, soaking, spraying or coating a medical device with the antimicrobial composition, as mentioned above and shown in the Examples below.

In another form, the antimicrobial agents described herein may be blended with the polymer or polymer mixture used to form the medical device and then subsequently molded or extruded to form the medical device. The antimicrobial agents described herein may be present in the polymer or polymer mixture in a concentration of from 1 to 15% w/w, or from 1.5 to 7.5% w/w, or from 2.0 to 5.0% w/w. In one form, the TCM antimicrobial agents disclosed herein are present in a concentration of about 2.5% w/w. In another form, the TCM antimicrobial agents disclosed herein are present in a concentration of about 5% w/w. As may be appreciated by those skilled in the art, a master batch of antimicrobial agent and polymer may first be formed and then blended with the balance of the polymer or polymer mixture to achieve the desired concentration of TCM antimicrobial agent.

As may be appreciated, medical devices typically are sterilized to render microorganisms located thereon substantially non-viable. In particular, sterile is understood in the field of art to mean a minimum sterility assurance level of 10⁻⁶. Examples of sterilization processes are described in U.S. Patent Nos. 3,815,315, 3,068,864, 3,767,362, 5,464,580, 5,128,101 and 5,868,244. Specifically, absorbable medical devices may be sensitive to radiation and heat. Accordingly, it may be desirable to sterilize such devices using conventional sterilant gases or agents, such as, for example, ethylene oxide gas.

Absorbable medical devices are sensitive to moisture and are therefore often packaged in hermetically sealed packages, such as sealed foil packages. However, sealed foil packages are also impervious to sterilant gas. In order to compensate for this and utilize foil packages in ethylene oxide gas sterilization processes, processes have been developed using foil packages having gas permeable or pervious vents (e.g., Tyvek® nonwoven material, manufactured by E. I. du Pont de Nemours and Company of Wilmington, Delaware). The gas permeable vents are mounted to an open end of the package and allow the passage of air, water vapor and ethylene oxide into the interior of the package. After the sterilization process is complete, the package is sealed adjacent to the vent so the vent is effectively excluded from the sealed package, and the vent is cut away or otherwise removed, thereby producing a gas impervious hermetically sealed package. Another type of foil package having a vent is a pouch-type package having a vent mounted adjacent to an end of the package, wherein the vent is sealed to one side of the package creating a vented section. After the sterilization process is complete the package is sealed adjacent to the vented section, and the sealed package is cut away for the vented section.

### EXAMPLES

### Test Method Used in Measurement of Sodium Houttuyfonate Content

As indicated above, the antimicrobial compositions for coating a medical device as disclosed herein, may include a polymeric film forming material; and an antimicrobial agent comprising a material selected from the group of extract of Houttuynia cordata, sodium houttuyfonate and sodium new houttuyfonate and mixtures thereof. In one form, the composition may also include calcium stearate, lactide and glycolide co-polymer. In order to accurately determine sodium houttuyfonate (SH) content for a coated medical device such as an absorbable suture, the following test method was developed.

The test methods previously available for SH content determinations include iodine titration and UV methods. When such methods are used to determine SH content on an absorbable suture they tend to interfere with the suture matrix and, as such, are ineffective for product quality control testing. For example, a basic solution is used when conducting the iodine titration method, which reacts with the suture polymer. In addition, tens of grams of suture samples are required for each test in order to reach the sensitivity of the titration method. The UV spectroscopic method is more sensitive; however the coating materials of can interfere with UV spectrometry detection.

As such, a test method for sodium houttuyfonate determination was developed that utilizes high performance liquid chromatography (HPLC). This method can be applied to both non-sterile and sterile absorbable medical devices, including sutures.

The method so developed includes two steps: (1) removal of sodium houttuyfonate from the absorbable medical device matrix in an appropriate solvent system and (2) detection and quantification of sodium houttuyfonate by HPLC with UV detection.

The solubility properties of sodium houttuyfonate indicate that it is only slightly soluble in cold water and most organic solvents, such as acetonitrile and methanol. Sodium houttuyfonate must be completely removed from the medical device coating, which, as indicated, may also contain calcium stearate and lactide/glycolide co-polymer, and be dissolved into the extraction solution for quantification. A mixed solvent system with solvents of different polarity index and appropriate pH was found to achieve the desired extraction efficiency. The solvent system consists of 70% pH 10.8 phosphate buffer water solution and 30% acetonitrile. The extraction procedure involves placing the medical device sample in the solvent and shaking the sample for 30 minutes at ambient temperature. The extraction efficiency analysis results indicate greater than 99% sodium houttuyfonate recovery.

The HPLC determinations, performed in the tests below, utilized an Agilent 1100 Series model with a Phenomenex, Gemini-NX C18, 250 x 4.6mm, 5µm HPLC column. A 70% of pH 10.8 phosphate buffer and 30% acetonitrile mixed solution was used as the mobile phase. The sodium houttuyfonate compound is detected by a UV detector at a λ=283 nm wavelength. The HPLC conditions were as follows:
Column: Gemini-NX, 5µm, 110A°, 4.6x250 mm, Phenomenex
Mobile Phase: CH₃CN: Buffer* 300: 700
   * 0.017M K₂HPO₄, pH=10.8 water solution
Flow: 2.0 ml/min
Temperature of column: 55°C
Injection volume: 10 µl
Run time: 8 min

### Example 1. The Making of a TCM Suture

An L(-) lactide/glycolide copolymer containing 65 mole% lactide and 35 mole% glycolide was dissolved in ethyl acetate at 4.5% w/w. Into the solution was added sodium houttuyfonate (Second Pharm Co LTD Lot# 071213) at 2.5% and 5% w/w. Calcium stearate was added to the solution at 4.5% w/w. The solution was vortexed at room temperature until an even suspension was formed. A 2/0 polyglactin 910 dyed braided suture was coated by immersing the suture into the suspension. The suture was dried at room temperature. The TCM coated suture was cut into 27 inch long and EO sterilized.

The TCM coated suture so made was evaluated for physical properties. The TCM suture passed the routine suture performance testing, which indicates a normal physical property of the prototype suture. The suture coated with sodium houttuyfonate as discussed above was designated as prototype TCM-1 suture.

### Example 2. Ethylene Oxide Sterilization Stability

The TCM-1 suture, prepared in Example 1, was analyzed by HPLC and in vitro efficacy assays before and after ethylene oxide (EO) sterilization. Results presented in Tables 3 and 4, below, indicate that the TCM coated suture has an acceptable stability for EO sterilization.

### Example 3.

The antimicrobial efficacy was evaluated in vitro by zone of inhibition assay and log reduction assay. Data in Table 1 and Table 2 indicate that the TCM-1 suture showed in vitro efficacy against methicillin-resistant Staphylococcus Aureus (MRSA) and methicillin-resistant Staphylococcus Epidermidis (MRSE) by zone of inhibition and log reduction assays.

Results from in vitro efficacy testing of the TCM-1 suture indicates that, while it is less effective than a commercially available antibacterial polyglactin 910 suture for a zone of inhibition assay, it is more effective than a commercially available antibacterial polyglactin 910 suture for a log reduction assay, suggesting a different mode of action. TCM-1 activity appears to be rapidly bactericidal for susceptible strains. This differs from the predominantly bacteriostatic activity of triclosan, which is employed with commercially available antibacterial polyglactin 910 sutures.

**Table 1. In vitro Efficacy by Zone of Inhibition (ZOI)**

| **Suture** | **Zone of Inhibition (mm)** | |
|---|---|---|
| | **MRSA** | **MRSE** |
| TCM-1 suture 2.5% | 1.6 | 1.8 |
| TCM-1 suture 5% | 2.4 | 3.0 |

**Table 2. In vitro Efficacy by Log Reduction**

| **Suture** | **Log reduction** | | |
|---|---|---|---|
| | **MRSA** | **MRSE** | ***E. coli*** |
| TCM-1 suture 2.5% | 4.0 | 4.0 | 0.7 |
| TCM-1 suture 5% | 4.0 | 4.0 | 1.0 |
| Antibacterial polyglactin 910 suture* | 1.7 | 2.0 | 0 |
| Control suture (Non-antibacterial polyglactin 910 suture*) | 0 | 0 | 0 |

| | | | |
|---|---|---|---|
| * Commercially available | | | |

**Table 3. Stability for EO Sterilization**

| **MRSA** | | |
|---|---|---|
| **Suture** | **Zone of inhibition (mm)** | **Log reduction** |
| TCM-1 suture 2.5% | 1.6 | 4.0 |
| TCM-1 suture 2.5% EO | 1.3 | 4.0 |
| TCM-1 suture 5% | 2.4 | 4.0 |
| TCM-1 suture 5% EO | 2.0 | 4.0 |

**Table 4. Effect of EO Sterilization and Storage on Houttuyfonate-Na Content in TCM-1 Suture**

| **Sodium Houttuyfonate Content** | | |
|---|---|---|
| **Suture sample** | **PPM** | ***% loss** |
| 2.5% TCM-1 suture Non-EO baseline | 1968 | N/A |
| 2.5% TCM-1 suture EO | 1685 | 14.4 |
| 5% TCM-1 suture Non-EO-baseline | 4282 | N/A |
| 5% TCM-1 suture EO | 3307 | 22.8 |

| | | |
|---|---|---|
| • % loss = (ppm of Non EO suture - ppm of EO suture/ ppm of Non EO suture 100%) | | |

Absorbable sutures were dip-coated in a mixture of a selected TCM, absorbable coating polymers and other additives in ethyl acetate. The coated sutures were dried, sterilized and packaged. The products were found to have desirable physical and mechanical properties and antibacterial/antimicrobial properties.

### Example 4. Effect of Particle Size and Particle Size Distribution on Coating Stability and Antimicrobial Efficacy

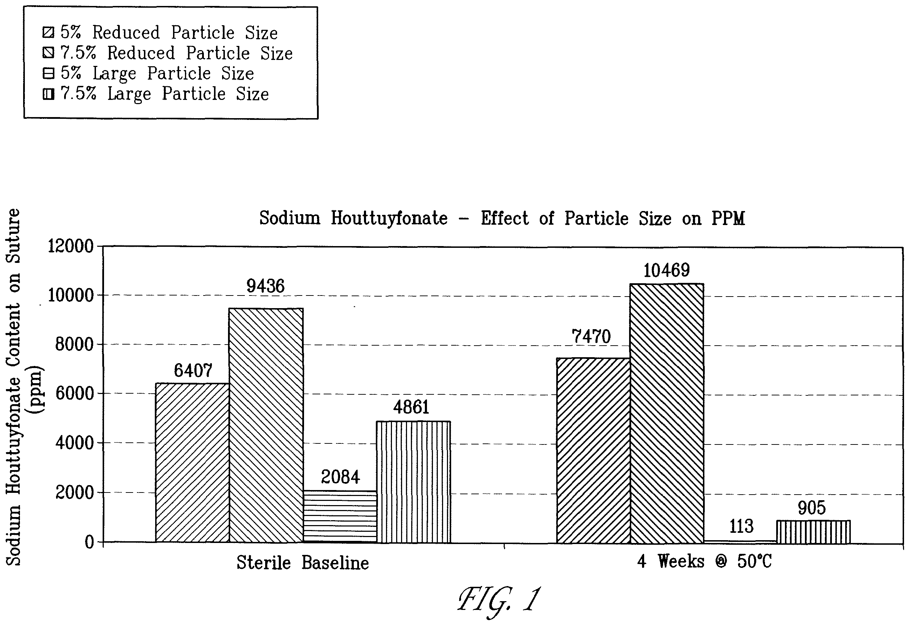

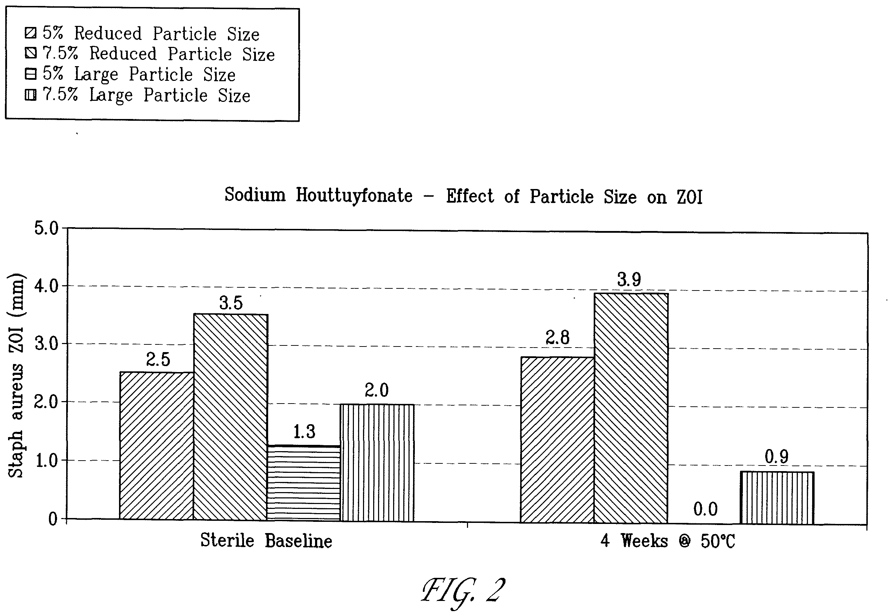

Studies were run using sutures coated with the sodium houttuyfonate antimicrobial coatings disclosed herein, with the sodium houttuyfonate coatings prepared using either ground or unground sodium houttuyfonate. The sutures were coated at two coating levels, using the coating method described hereinabove. Particle size analysis was performed using a Beckman Coulter model LS 320 laser diffraction particle size analyzer. Results for these studies are presented below in Tables 5-7 and graphically depicted in FIGS. 1 and 2.

As indicated below, coated sutures employing ground sodium houttuyfonate were found to yield a higher efficiency during coating, more sodium houttuyfonate on a suture for the same amount of sodium houttuyfonate in the coating dispersion, a much more consistent level of sodium houttuyfonate on the suture and a significant reduction in percent RSD. Further, the ground sodium houttuyfonate did not show significant loss of concentration (PPM) or reduction in zone of inhibition (ZOI), when stored at 25 and 50°C for four weeks. Ground sodium houttuyfonate provided larger Staph aureus zones of inhibition after ethylene oxide (EO) sterilization. Ground sodium houttuyfonate was found to yield a larger Staph aureus Log Reduction after EO sterilization and did not show a significant reduction in Log Reduction when stored at 25 and 50°C for four weeks.

**Table 5**

| **Effect of Particle Size and Coating Level on Performance for Sodium Houttuyfonate (SH)** | | | |
|---|---|---|---|
| Description | Unground SH (As received from vendor) | Ground SH (Reduced particle size) | Benefits of Reduced Particle Size Sodium Houttuyfonate |
| Mean Particle Size (µm) | 144.7 | 37.4 | |
| Standard Deviation Particle Size (µm) | 140.1 | 25.8 | |
| % Particles Below 100 µm | 50.5 | 99.4 | |
| % Particles Below 10 µm | 1.8 | 18.2 | |
| | | | |
| SH PPM Content for Sterile Baseline 5% Coating | 2084 | 6407 | Ground SH yields a higher efficiency during coating - more SH on suture for same amount in coating dispersion |
| SH PPM Content for Sterile Baseline 7.5% Coating | 4861 | 9436 | |
| | | | |
| SH PPM %RSD for Sterile Baseline 5% Coating | 20.6 | 0.9 | Ground SH produces much more consistent levels on suture - %RSD is significantly reduced |
| SH PPM %RSD for Sterile Baseline 75% Coating | 17.4 | 0.2 | |
| | | | |
| SH PPM Content after 4 weeks at 50°C for 5% Coating | 113 | 7470 | Ground SH does not show significant loss of PPM when stored at 50°C for four weeks |
| SH PPM Content after 4 weeks at 50°C for 7.5% Coating | 905 | 10469 | |
| | | | |
| Staph aureus ZOI for Sterile Baseline 5% Coating | 1.3 | 2.5 | Ground SH shows larger Staph aureus zones of inhibition after EO sterilization |
| Staph aureus ZOI for Sterile Baseline 7.5% Coating | 2.0 | 3.5 | |
| Staph aureus ZOI after 4 weeks at 50°C for 5% Coating | 0.2 | 28 | Ground SH does not show significant reduction in ZOI when stored at 50°C for four weeks |
| Staph aureus ZOI after 4 weeks at 50°C for 7.5% Coating | 1.0 | 3.9 | |
| | | | |
| Staph aureus Log Reduction for Sterile Baseline 5% Coating | 1.1 | 3.8 | Ground SH shows larger Staph aureus Log Reduction after EO sterilization |
| Staph aureus Log Reduction for Sterile Baseline 7.5% Coating | 3.6 | 3.8 | |
| | | | |
| Staph aureus Log Reduction after 4 weeks at 50°C for 5% Coating | 0.2 | 3.6 | Ground SH does not show significant reduction in Log Reduction when stored at 50°C for four weeks |
| Staph aureus Log Reduction after 4 weeks at 50°C for 7.5% Coating | 1.0 | 3.6 | |

**Table 6**

| **Effect of Particle Size, Coating Level and Storage Conditions** | | | | | | | | | | | | | | | |
|---|---|---|---|---|---|---|---|---|---|---|---|---|---|---|---|
| | | Laboratory Ground SH | | | | | | | Unground SH | | | | | | |
| | | SH Suture Content (ppm) | | | | | | | SH Suture Content (ppm) | | | | | | |
| Coating Level (%) | Storage Condition | 1 | 2 | 3 | AVE | SD | % RSD | % Change | 1 | 2 | 3 | AVE | SD | % RSD | % Change |
| 5.0 | Sterile Baseline | 6477 | 6371 | 6374 | 6407 | 60 | 0.9% | | 1811 | 2579 | 1862 | 2084 | 429 | 20.6% | - |
| 7.5 | Sterile Baseline | 9442 | 9416 | 9450 | 9436 | 18 | 0.2% | | 5809 | 4178 | 4597 | 4861 | 847 | 17.4% | - |
| 5.0 | 4 Weeks @ 25°C | 8813 | 8088 | 7935 | 8279 | 469 | 5.7% | 29% | 2640 | 2073 | 2112 | 2275 | 317 | 13.9% | 9% |
| 7.5 | 4 Weeks @ 25°C | 11817 | 10919 | 11116 | 11284 | 472 | 4.2% | 20% | 4161 | 2382 | 2184 | 2909 | 1089 | 37.4% | -40% |
| 5.0 | 4 Weeks @ 50°C | 7614 | 7309 | 7486 | 7470 | 153 | 2.1% | 17% | 223 | 57 | 60 | 113 | 95 | 83.8% | -95% |
| 7.5 | 4 Weeks @ 50°C | 10701 | 10587 | 10119 | 10469 | 308 | 2.9% | 11% | 284 | 1170 | 1262 | 905 | 540 | 59.7% | -81% |

**Table 7**

| **Effect of Particle Size, Coating Level and Storage Conditions** | | | | | | | | | | | | | | | |
|---|---|---|---|---|---|---|---|---|---|---|---|---|---|---|---|
| | | Laboratory Ground SH | | | | | | | Unground SH | | | | | | |
| | | Microbiology Data | | | | | | | Microbiology Data | | | | | | |
| Coating Level (%) | Storage Condition | SA 1 | SA 2 | SA 3 | ZOI AVE | ZOI SD | SA LR | EC LR | SA 1 | SA 2 | SA 3 | ZOI AVE | ZOI SD | SA LR | EC LR |
| 5.0 | Sterile Baseline | 2.4 | 2.7 | 2.5 | 25 | 0.2 | 3.8 | 0.3 | | | | 1.3 | - | 1.1 | - |
| 7.5 | Sterile Baseline | 3.5 | 3.6 | 3.5 | 3.5 | 0.1 | 3.8 | 0.3 | | | | 2.0 | - | 3.6 | - |
| 5.0 | 4 Weeks @ 25°C | 3.6 | 3.6 | 3.5 | 3.6 | 0.1 | 3.6 | - | | | | 1.6 | | 1.6 | |
| 7.5 | 4 Weeks @ 25°C | 3.6 | 4.1 | 3.8 | 3.8 | 0.3 | 3.6 | - | | | | 22 | | 3.6 | |
| 5.0 | 4 Weeks @ 50°C | 2.8 | 2.9 | 2.8 | 2.8 | 0.1 | 3.6 | - | | | | 0.0 | | 0.2 | |
| 7.5 | 4 Weeks @ 50°C | 3.9 | 4 | 3.9 | 3.9 | 0.1 | 3.6 | - | | | | 0.9 | | 1.0 | |

The present invention includes a medical device as defined in claim 1 having an antimicrobial composition comprising an antimicrobial agent selected from the group of extract of Houttuynia cordata, sodium houttuyfonate, sodium new houttuyfonate and mixtures thereof. In one form the antimicrobial agent comprises sodium new houttuyfonate. In another form, the antimicrobial agent comprises sodium houttuyfonate. In another form, the sodium houttuyfonate or sodium new houttuyfonate has a mean particle size of less than 100 µm. In another form, the antimicrobial agent has a mean particle size of less than 50 µm. In another form, the antimicrobial agent has a mean particle size of less than 40 µm. In another form, the antimicrobial agent has a mean particle size of less than 40 µm and a standard deviation particle size of less than 30 µm. In another form, the antimicrobial composition is coated onto at least one surface of the medical device and further comprises a polymeric film forming material. In one form, the polymeric film forming material comprises a biocompatible, biodegradable polymer, copolymer or blend thereof. In another form, the antimicrobial agent is present in a concentration of from 1 to 15% w/w. In another form, the antimicrobial agent is present in a concentration of from 1.5 to 7.5% w/w. In another form, the antimicrobial agent is present in a concentration of from 2.0 to 5.0% w/w. In one form, the antimicrobial agent has a mean particle size effective to enhance the antimicrobial efficacy of the antimicrobial agent. In another form, the medical device is in the form of a fiber, mesh, powder, microspheres, flakes, sponge, foam, fabric, nonwoven, woven mat, a film, suture anchor device, suture, catheter, staple, stent, surgical tack, clips, plate and screw, drug delivery device, adhesion prevention barrier, and tissue adhesive.

A method as defined in the accompanying claims of making an antimicrobial medical device, the method comprising the step of applying an antimicrobial coating to the medical device, the antimicrobial coating including a polymeric film forming material; and an antimicrobial agent, the antimicrobial agent including a material selected from the group of extract of Houttuynia cordata, sodium houttuyfonate, sodium new houttuyfonate and mixtures thereof. In one form, the antimicrobial agent comprises sodium new houttuyfonate. In another form, the antimicrobial agent comprises sodium houttuyfonate. In another form, the antimicrobial agent has a mean particle size of less than 100 µm. In another form, the antimicrobial agent has a mean particle size of less than 50 µm. In another form, the antimicrobial agent has a mean particle size of less than 40 µm. The antimicrobial agent is present in a concentration of from 1 to 15% w/w. In another form, the antimicrobial agent is present in a concentration of from 1.5 to 7.5% w/w. In another form, the method further comprises the step of sterilizing the antimicrobial medical device.

## Claims

1. A medical device comprising an antimicrobial composition, wherein said medical device is in the form of a suture anchor device, suture, catheter, staple, stent, surgical tack, clips, plate and screw, adhesion prevention barrier, or tissue adhesive, and **characterized in that** said antimicrobial composition comprises an antimicrobial agent selected from the group of extract of Houttuynia cordata, sodium houttuyfonate, sodium new houttuyfonate and mixtures thereof.

2. The medical device of claim 1, wherein the antimicrobial agent has a mean particle size of less than 40µm and a standard deviation particle size of less than 30µm.

3. The medical device of claim 1, wherein the antimicrobial composition is in the form of a coating on at least one surface of the medical device and said coating further comprises a polymeric film forming material.

4. The medical device according to claim 3, wherein the antimicrobial agent comprises sodium new houttuyfonate.

5. The medical device according to claim 3, wherein the antimicrobial agent comprises sodium houttuyfonate.

6. The medical device according to any of claims 3 to 5, wherein the antimicrobial agent has a mean particle size of less than 100 µm.

7. The medical device according to claim 6, wherein the antimicrobial agent has a mean particle size of less than 50 µm.

8. The medical device according to claim 6, wherein the antimicrobial agent has a mean particle size of less than 40µm.

9. The medical device according to any of claims 3 to 8, wherein the polymeric film forming material comprises a biocompatible, biodegradable polymer, copolymer or blend thereof.

10. The medical device according to any of claims 3 to 8, wherein the polymeric film forming material is selected from aliphatic polyesters, poly(amino acids), copoly(ether-esters), polyalkylenes oxalates, polyamides, poly(ethylene glycol), poly(iminocarbonates), polyorthoesters, polyoxaesters, polyamidoesters, polyoxaesters containing amine groups, poly(anhydrides), polyphosphazene, polysaccharide gels and copolymers and blends thereof.

11. The medical device according to claim 10, wherein the polymeric film forming material is selected from homopolymers and copolymers of lactide, glycolide, epsilon-caprolactone, para-dioxanone, trimethylene carbonate, alkyl derivatives of trimethylene carbonate, monoglyceride polyesters, carboxymethyl cellulose hydrogels and blends thereof.

12. The medical device according to any of claims 3 to 11, wherein said coating further comprises calcium stearate.

13. The medical device according to any of claims 3 to 12, wherein said device is a suture.

14. A method of making an antimicrobial medical device as claimed in any one of claims 3 to 13, the method comprising the step of applying an antimicrobial coating composition to the medical device, the antimicrobial coating composition including a polymeric film forming material and from 1% to 15% w/w of an antimicrobial agent selected from the group of sodium houttuyfonate, sodium new houttuyfonate and mixtures thereof.

## Patentansprüche

1. Medizinische Vorrichtung, die eine antimikrobielle Zusammensetzung umfasst, wobei die medizinische Vorrichtung in Form von einer Nahtankervorrichtung, eines Nahtmaterials, eines Katheters, einer Heftklammer, eines Stents, einer chirurgischen Klammer, Klemmen, einer Platte und Schraube, einer Adhäsionsverhinderungsbarriere oder eines Gewebeklebers vorliegt, und die **dadurch gekennzeichnet ist, dass** die antimikrobielle Zusammensetzung ein antimikrobielles Mittel umfasst, das aus der Gruppe von Houttuynia-cordata-Extrakt, Natrium-Houttuyfonat, Natrium-Neo-Houttuyfonat und Mischungen davon ausgewählt ist.

2. Medizinische Vorrichtung gemäß Anspruch 1, wobei das antimikrobielle Mittel eine mittlere Teilchengröße von weniger als 40 µm und eine Standardabweichungs-Teilchengröße von weniger als 30 µm aufweist.

3. Medizinische Vorrichtung gemäß Anspruch 1, wobei die antimikrobielle Zusammensetzung in Form einer Beschichtung auf mindestens einer Oberfläche der medizinischen Vorrichtung vorliegt und die Beschichtung ferner ein polymeres filmbildendes Material umfasst.

4. Medizinische Vorrichtung gemäß Anspruch 3, wobei das antimikrobielle Mittel Natrium-Neo-Houttuyfonat umfasst.

5. Medizinische Vorrichtung gemäß Anspruch 3, wobei das antimikrobielle Mittel Natrium-Houttuyfonat umfasst.

6. Medizinische Vorrichtung gemäß beliebigen der Ansprüche 3 bis 5, wobei das antimikrobielle Mittel eine mittlere Teilchengröße von weniger als 100 µm aufweist.

7. Medizinische Vorrichtung gemäß Anspruch 6, wobei das antimikrobielle Mittel eine mittlere Teilchengröße von weniger als 50 µm aufweist.

8. Medizinische Vorrichtung gemäß Anspruch 6, wobei das antimikrobielle Mittel eine mittlere Teilchengröße von weniger als 40 µm aufweist.

9. Medizinische Vorrichtung gemäß beliebigen der Ansprüche 3 bis 8, wobei das polymere filmbildende Material ein biokompatibles, biologisch abbaubares Polymer, Copolymer oder ein Gemisch davon umfasst.

10. Medizinische Vorrichtung gemäß beliebigen der Ansprüche 3 bis 8, wobei das polymere filmbildende Material aus aliphatischen Polyestern, Poly(aminosäuren), Copoly(etherestern), Polyalkylen-oxalaten, Polyamiden, Poly(ethylenglycol), Poly(iminocarbonaten), Polyorthoestern, Polyoxaestern, Polyamidoestern, aminogruppenhaltigen Polyoxaestern, Poly(anhydriden), Polyphosphazen, Polysaccharidgelen und Copolymeren und Gemischen davon ausgewählt ist.

11. Medizinische Vorrichtung gemäß Anspruch 10, wobei das polymere filmbildende Material aus Homopolymeren und Copolymeren von Lactid, Glycolid, Epsilon-Caprolacton, Para-Dioxanon, Trimethylencarbonat, Alkylderivaten von Trimethylencarbonat, Monoglyceridpolyestern, Carboxymethylcellulose-Hydrogelen und Gemischen davon ausgewählt ist.

12. Medizinische Vorrichtung gemäß beliebigen der Ansprüche 3 bis 11, wobei die Beschichtung ferner Calciumstearat umfasst.

13. Medizinische Vorrichtung gemäß beliebigen der Ansprüche 3 bis 12, wobei die Vorrichtung ein Nahtmaterial ist.

14. Verfahren zur Herstellung einer antimikrobiellen medizinischen Vorrichtung, wie in einem der Ansprüche 3 bis 13 beansprucht, wobei das Verfahren den Schritt des Aufbringens einer antimikrobiellen Beschichtungszusammensetzung auf die medizinische Vorrichtung umfasst, wobei die antimikrobielle Beschichtungszusammensetzung ein polymeres filmbildendes Material und 1% bis 15% Gew./Gew. eines antimikrobiellen Mittels einschließt, das aus der Gruppe von Natrium-Houttuyfonat, Natrium-Neo-houttuyfonat und Mischungen davon ausgewählt ist.

## Revendications

1. Dispositif médical comprenant une composition antimicrobienne, ledit dispositif médical étant sous la forme d'un dispositif d'ancrage de suture, d'une suture, d'un cathéter, d'une agrafe, d'un stent, d'une colle chirurgicale, de pinces, d'une plaque et de vis, d'une barrière anti-adhérences ou d'un adhésif tissulaire, et **caractérisé en ce que** ladite composition antimicrobienne comprend un agent antimicrobien sélectionné dans le groupe constitué d'un extrait de Houttuynia cordata, du houttuyfonate de sodium, du nouveau houttuyfonate de sodium, et de mélanges de ceux-ci.

2. Dispositif médical selon la revendication 1, dans lequel l'agent antimicrobien a une taille de particule moyenne inférieure à 40 µm et une taille de particule correspondant à l'écart-type inférieure à 30 µm.

3. Dispositif médical selon la revendication 1, dans lequel la composition antimicrobienne est sous la forme d'un revêtement sur au moins une surface du dispositif médical et ledit revêtement comprend en outre un matériau formant un film polymère.

4. Dispositif médical selon la revendication 3, dans lequel l'agent antimicrobien comprend du nouveau houttuyfonate de sodium.

5. Dispositif médical selon la revendication 3, dans lequel l'agent antimicrobien comprend du houttuyfonate de sodium.

6. Dispositif médical selon l'une quelconque des revendications 3 à 5, dans lequel l'agent antimicrobien a une taille de particule moyenne inférieure à 100 µm.

7. Dispositif médical selon la revendication 6, dans lequel l'agent antimicrobien a une taille de particule moyenne inférieure à 50 µm.

8. Dispositif médical selon la revendication 6, dans lequel l'agent antimicrobien a une taille de particule moyenne inférieure à 40 µm.

9. Dispositif médical selon l'une quelconque des revendications 3 à 8, dans lequel le matériau formant un film polymère comprend un polymère ou un copolymère biocompatible, biodégradable, ou un mélange de ceux-ci.

10. Dispositif médical selon l'une quelconque des revendications 3 à 8, dans lequel le matériau formant un film polymère est sélectionné parmi des polyesters aliphatiques, des poly(acides aminés), des copoly(étheresters), des oxalates de polyalkylène, des polyamides, le poly(éthylène glycol), des poly(iminocarbonates), des polyorthoesters, des polyoxaesters, des polyamidoesters, des polyoxaesters contenant des groupes amine, des poly(anhydrides), le polyphosphazène, des gels de polysaccharides, et des copolymères et mélanges de ceux-ci.

11. Dispositif médical selon la revendication 10, dans lequel le matériau formant un film polymère est sélectionné parmi des homopolymères et copolymères de lactide, de glycolide, d'epsilon-caprolactone, de para-dioxanone, de carbonate de triméthylène, de dérivés alkyles de carbonate de triméthylène, de polyesters monoglycéridiques, d'hydrogels de carboxyméthylcellulose, et de mélanges de ceux-ci.

12. Dispositif médical selon l'une quelconque des revendications 3 à 11, dans lequel ledit revêtement comprend en outre du stéarate de calcium.

13. Dispositif médical selon l'une quelconque des revendications 3 à 12, dans lequel ledit dispositif est une suture.

14. Procédé de fabrication d'un dispositif médical antimicrobien selon l'une quelconque des revendications 3 à 13, le procédé comprenant l'étape d'application d'une composition de revêtement antimicrobien sur le dispositif médical, la composition de revêtement antimicrobien comprenant un matériau formant un film polymère et de 1 % à 15 % p/p d'un agent antimicrobien sélectionné dans le groupe constitué du houttuyfonate de sodium, du nouveau houttuyfonate de sodium, et de mélanges de ceux-ci.
